# EUROPEAN PATENT SPECIFICATION

(11) **EP 0 639 180 B1**
(45) Date of publication and mention of the grant of the patent: **05.07.2000**
(21) Application number: 93908004.0
(22) Date of filing: 06.04.1993
(51) Int. Cl.: C07D 211/90, C07D 405/12

(54) **PROCESS FOR STEREOSPECIFIC HYDROLYSIS OF PIPERIDINEDIONE DERIVATIVES**
VERFAHREN ZUR STEREOSPEZIFISCHEN HYDROLYSE VON PIPERIDIN-DION DERIVATEN
PROCEDE D'HYDROLYSE STEREOSPECIFIQUE DE DERIVES DE PIPERIDINEDIONE

(30) Priority: 06.05.1992 GB 9209687
(43) Date of publication of application: 22.02.1995
(73) Proprietor: SMITHKLINE BEECHAM PLC, Brentford, Middlesex TW8 9EP (GB)
(72) Inventor: CURZONS, Alan David, Worthing West Sussex BN14 8QH (GB); POWELL, Lawson William, Worthing West Sussex BN14 8QH (GB); KEAY, Alan Martin, Worthing West Sussex BN14 8QH (GB)
(74) Representative: Russell, Brian John
(86) International application number: GB9300721
(87) International publication number: WO9322284

(56) References cited:
- EP-A- 0 223 334
- EP-A- 0 223 403
- EP-A- 0 374 675
- US-A- 4 007 196

## Description

The present invention is concerned with a new process and certain novel intermediates.

US Patent No. 4,007,196 describes a class of compounds which possess anti-depressant activity. One specific compound mentioned in the patent is paroxetine which is described as possessing anti-depressant activity.

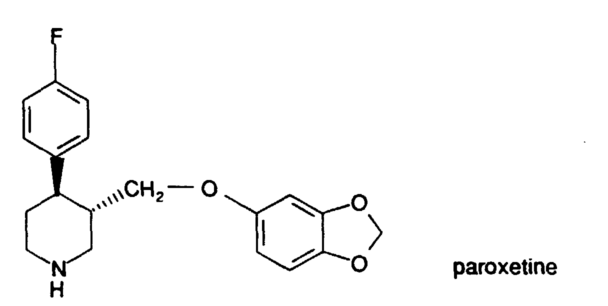

This compound has now been approved for human use in some countries and is being sold as an anti-depressant agent.

All described processes for preparing paroxetine involve chemical reactions such as those described in US Patent 4902801. It will be appreciated that paroxetine is actually the (-) isomer (as shown above) and that all chemical methods of preparing paroxetine involve a chemical resolution step which wastes substrate and reactants and necessitates the use of expensive resolving agents and is a fairly expensive reaction to perform.

The present invention involves the use of an enzymatic resolution step which allieviates or overcomes a number of problems associated with a purely chemical resolution step.

Accordingly, the present invention provides a process for stereospecifically hydrolysing a mixture of the (+) and (-) isomers of a compound of formula (II): 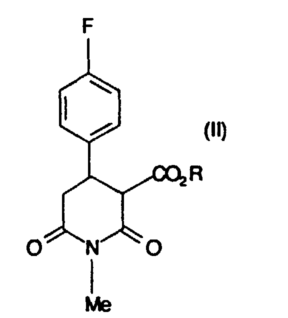 in which R is C₁₋₆ alkyl; using a carboxyl esterase enzyme,
(i) to form a compound of formula (IIIA), 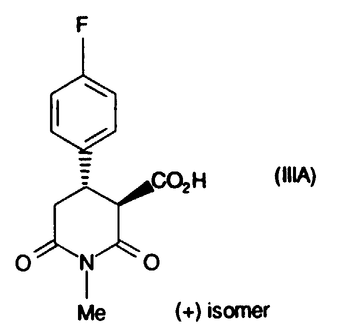 and thereafter separating the resulting compound of formula (IIIA) from the remaining (-) isomer of formula (II); or
ii) to form a compound of formula (IIIB): 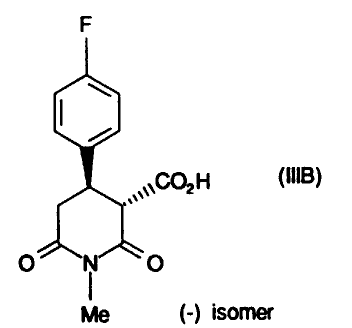 and thereafter separating the resulting compound of formula (IIIB) from the remaining (+) isomer of formula (II).

It should be appreciated that the choice of carboxy esterase enzyme will determine which isomer of formula (II) is hydrolysed to the corresponding acid, which may be determined by routine experimentation.

Process variant i) is preferred.

When using process variant (i) the stereoselectivity of the process is such that from a racemic mixture of a compound of formula (II), after the action of the carboxyl esterase enzyme the ratio of (-) to (+) isomer of formula (II), is greater than 60%, preferably greater than 70%, more preferably greater than 80% and most preferably greater than 85%.

When using process variant (ii) the stereoselectivity of the process is such that from a racemic mixture of a compound of formula (II), after the action of the carboxy esterone enzyme, the ratio of (+) to (-) isomer of a compound of formula (II), is greater than 60%, preferably greater than 70%, more preferably greater than 80% and most preferably greater than 85%.

The process is suitably carried out by dissolving the (±) unresolved compound of formula (II) into a suitable solvent such as an aqueous/organic solvent mixture and adding the carboxyl esterase enzyme and stirring the resulting mixture until the reaction is completed.

Suitable temperatures for performing the reaction include 0-50°C more suitably 10-40°C and yet more suitably 25 to 35°C and most suitably at 30°C.

Suitable aqueous/organic solvent mixtures include buffered aqueous solvents such as tris buffer which is mixed with DMSO.

Suitable pH's for the reaction to be carried out and include pH 4 to 8, more suitably pH 5 to 7 and preferably at pH 5.5.

Suitable values for the variable R include methyl and ethyl. Preferably R is ethyl.

The term carboxyl esterase enzyme is internationally recognised as being those enzymes which fall within the class EC 3.1.1.

Suitable carboxyl esterase enzymes include Porcine liver esterase and Bovine liver esterase both of which are commercially available or may be extracted from Porcine liver or Bovine liver respectively by using procedures available in the literature.

It should also be appreciated that carboxyl esterase enzymes produced by microbes are also suitable for use in the present invention.

In process variant i) where the carboxyl esterase enzyme stereospecifically hydrolyses the (+) form of a compound of formula (II), the remaining (-) form of a compound of formula (II) is separated by conventional techniques such as solvent extraction of the compound of formula (II) using a non-aqueous miscible solvent such as ethyl acetate and the resulting (-) compound of formula (II) may then be isolated using conventional techniques such as precipitation.

The present invention also extends to a process for subsequently converting the (-) compound of formula (II) prepared as described above to paroxetine or a pharmaceutically acceptable salt and/or solvate thereof such as the hydrochloride hemi-hydrate, for example, using the procedures outlined in US Patent No. 4,902,801 and US Patent 4,721,723.

In process variant ii) where the carboxyl esterase enzyme stereospecifically hydrolyses the (-) form of a compound of formula (II) to yield the (-) form of a compound of formula (III), the remaining (+) form of a compound of formula (II) may be separated from the (-) form of a compound of formula (III) as mentioned above.

The (-) compound of formula (III) may be converted to paroxetine by first converting it to a (-) compound of formula (II) using conventional esterification techniques. The ester may then be convened to paroxetine or a pharmaceutically acceptable salt and/or solvate thereof such as the hydrochloride hemi-hydrate, for example, using the procedures outlined in US Patent No. 4,902,801 and US Patent 4,721,723.

Alternatively, the (-) compound of formula (III) may be directly convened to paroxetine by reducing the carboxylic add group to a hydroxymethyl group and reducing the two keto groups in the piperidine ring using conventional reducing agents such as lithium aluminium hydride. Subsequently, the resulting piperidine carbinol compound may be convened to paroxetine or a pharmaceutically acceptable salt and/or solvate thereof such as the hydrochloride hemi-hydrate, for example, using the procedures outlined in US Patent No. 4,902,801 and US Patent 4,721,723.

It is believed that both the (-) and (+) forms of a compound of formula (III) are novel as are any mixtures thereof including the racemate. The present invention also extends to a compound of formula (III) or a salt or hydrate thereof, the (-) and (+) forms and any mixtures thereof including the racemate.

Compounds of formula (II) may be prepared according to the procedures outlined in US Patent 4,902,801.

The present invention is illustrated by the following example.

### Example 1

### (-) trans-3-Ethoxycarbonyl-4-(4'-fluorophenyl)-N-methyl piperidine-2,6-dione

A solution of (±) trans-3-Ethoxycarbonyl-4-(4'-fluorophenyl)-N-methyl piperidine-2,6-dione (1.51g 5.15mmol) in DMSO (100ml) was added to Tris buffer (900ml, 0.2m, pH 5.5). The pH was readjusted to 5.5, pig liver esterase (Sigma Chemical Co, 19ml, 5340 units) added and the reaction stirred for twenty hours. The pH was maintained by addition of aqueous sodium hydroxide (0.105m, 25ml, 2.63mmol).

The reaction mixture was extracted with ether (3 x 300ml), the combined organic extracts washed with Tris buffer (0.1M, pH 8.5, 2 x 250ml), the Tris buffer extracts washed with ether (1 x 200ml) and the combined organic extracts dried over anhydrous magnesium sulphate.

The reaction mixture was assayed at 16, 18 and 20 hours. On each occasion the enantiomeric ratio was 90:10 (-):(+).

The solution evaporated to an oil and replaced with toluene/THF. This solution was then reduced with lithium aluminium hydride. The final solution containing a 0.15g of reduced material in 30ml THF/toluene had a rotation of -16.5°. Chiral HPLC indicated an isomer ratio of 86:14 (-):(+).

## Claims

1. A process for stereospecifically hydrolysing a mixture of the (+) and
(-) isomers of a compound of formula (II): 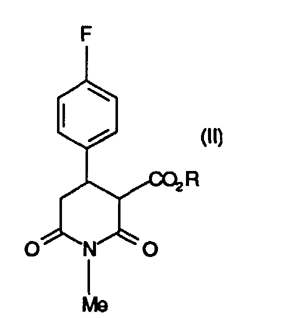 in which R is C₁₋₆ alkyl; using a carboxyl esterase enzyme.
(i) to form a compound of formula (IIIA), 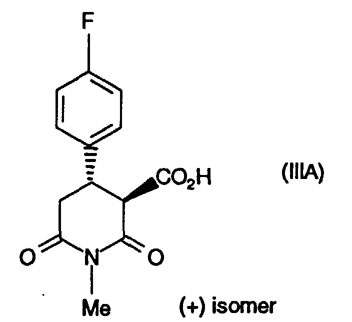 and thereafter separating the resulting compound of formula (IIIA) from the remaining (-) isomer of formula (II); or
ii) to form a compound of formula (IIIB): 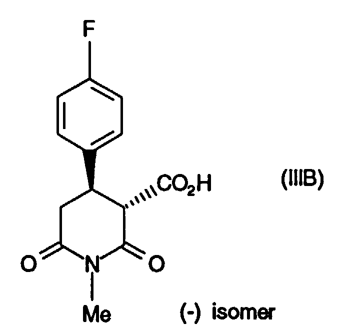 and thereafter separating the resulting compound of formula (IIIB) from the remaining (+) isomer of formula (II).

2. A process according to variant (i) in claim 1 in which the stereoselectivity of the process is such that from a racemic mixture of a compound of formula (II), after the action of the carboxyl esterase enzyme, the ratio of (-) to (+) isomer of formula (II), is greater than 60%.

3. A process according to variant (ii) in claim 1 in which the stereoselectivity of the process is such that from a racemic mixture of a compound of formula (II), after the action of the carboxy esterone enzyme, the ratio of (+) to (-) isomer of a compound of formula (II), is greater than 60%.

4. A process according to any one of claims 1 to 3 which is carried out by dissolving (±) unresolved compound of formula (II) into an aqueous/organic solvent mixture and adding the carboxyl esterase enzyme and stirring the resulting mixture until the reaction is completed.

5. A process according to any one of claims 1 to 4 in which the temperature for performing the reaction is 0-50°C.

6. A process according to any one of claim 1 to 5 in which the pH of the reaction is pH 4 to 8.

7. A process according to any one of claims 1 to 6 in which the variable R in formula (II) is methyl or ethyl.

8. A process according to claim 1 in which the carboxyl esterase enzyme is Porcine liver esterase or Bovine liver esterase.

9. A process for subsequently converting a (-) compound of formula (II), prepared as described in claim 1, to paroxetine or a pharmaceutically acceptable salt and/or solvate thereof.

10. A process for subsequently converting a (-) compound of formula (III), prepared as described in claim 1, to paroxetine by first converting it to a (-) compound of formula (II), as defined in claim 1, using conventional esterification techniques followed by subsequent convertion to paroxetine or a pharmaceutically acceptable salt and/or solvate thereof.

11. A process for subsequently converting a (-) compound of formula (III) directly to paroxetine by reducing the carboxylic acid group to a hydroxymethyl group and reducing the two keto groups in the piperidine ring and subsequently, converting the resulting piperidine carbinol to paroxetine or a pharmaceutically acceptable salt and/or solvate thereof.

12. A compound of formula (IIIA) or (IIIB) according to claim 1, or a salt or hydrate thereof.

## Patentansprüche

1. Verfahren zur stereospezifischen Hydrolyse eines Gemisches des (+)- und (-)-Isomers einer Verbindung der Formel (II): 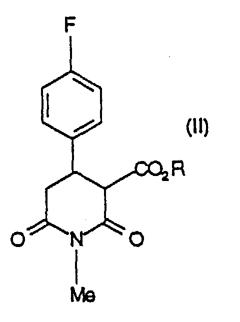 wobei R ein C₁₋₆ Alkylrest ist; unter Verwendung eines Carboxylesterase-Enzyms,
(i) zur Erzeugung einer Verbindung der Formel (IIIA), 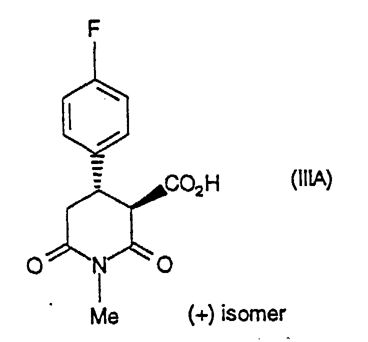 und danach Trennung der resultierenden Verbindung der Formel (IIIA) vom zurückbleibenden (-)-Isomer der Formel (II); oder
(ii) zur Erzeugung einer Verbindung der Formel (IIIB): 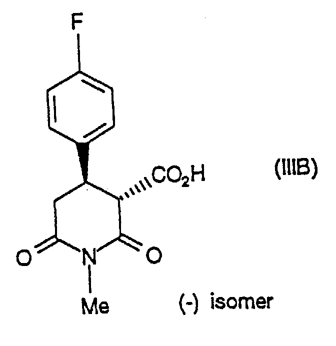 und danach Trennung der resultierenden Verbindung der Formel (IIIB) vom zurückbleibenden (+)-Isomer der Formel (II).

2. Verfahren nach Variante (i) in Anspruch 1, in welchem die Stereoselektivität des Verfahrens so ist, daß in einem racemischen Gemisch einer Verbindung der Formel (II) nach dem Wirken des Carboxylesterase-Enzyms das Verhältnis des (-)- zum (+)-Isomer der Formel (II) größer ist als 60%.

3. Verfahren nach Variante (ii) in Anspruch 1, in welchem die Stereoselektivität des Verfahrens so ist, daß in einem racemischen Gemisch einer Verbindung der Formel (II) nach dem Wirken des Carboxylesterase-Enzyms das Verhältnis des (+)- zum (-)-Isomer der Formel (II) größer ist als 60%.

4. Verfahren nach einem der Ansprüche 1 bis 3, das durch Auflösen von (±) nicht getrennter Verbindung der Formel (II) in einem wäßrigen/organischen Lösungsmittelgemisch und Hinzufügen des Carboxylesterase-Enzyms und Rühren des resultierenden Gemisches durchgeführt wird, bis die Reaktion vollständig ist.

5. Verfahren nach einem der Ansprüche 1 bis 4, in welchem die Temperatur für die Durchführung der Reaktion 0 bis 50°C beträgt.

6. Verfahren nach einem der Ansprüche 1 bis 5, in welchem der pH-Wert der Reaktion von pH 4 bis 8 beträgt.

7. Verfahren nach einem der Ansprüche 1 bis 6, in welchem der variable Rest R in Formel (II) eine Methyl- oder Ethylgruppe ist.

8. Verfahren nach Anspruch 1, in welchem das Carboxylesterase-Enzym Schweineleber-Esterase oder Rinderleber-Esterase ist.

9. Verfahren zum nachfolgenden Konvertieren einer (-)-Verbindung der Formel (II), die wie in Anspruch 1 beschrieben hergestellt wurde, zu Paroxetin oder einem pharmazeutisch verträglichem Salz und/oder Solvat davon.

10. Verfahren zum nachfolgenden Konvertieren einer (-)-Verbindung der Formel (III), die wie in Anspruch 1 beschrieben hergestellt wurde, zu Paroxetin, zuerst durch Konvertieren in eine (-)-Verbindung der Formel (II), wie in Anspruch 1 definiert, unter Verwendung von herkömmlichen Verestemngsmethoden, und nachfolgend Überführen in Paroxetin oder ein pharmazeutisch verträgliches Salz und/oder Solvat davon.

11. Verfahren zum nachfolgenden Konvertieren einer (-)-Verbindung der Formel (III) direkt zu Paroxetin durch Reduzieren der Carboxylgruppe zu einer Hydroxymethylgruppe und Reduzieren der zwei Ketogruppen im Piperidinring und nachfolgendes Überführen des resultierenden Piperidincarbinols zu Paroxetin oder einem pharmazeutisch verträglichem Salz und/oder Solvat davon.

12. Verbindung der Formel (IIIA) oder (IIIB) nach Anspruch 1 oder ein Salz oder Hydrat davon.

## Revendications

1. Procédé pour l'hydrolyse stéréospécifique d'un mélange des isomères (+) et (-) d'un composé de formule (II) : 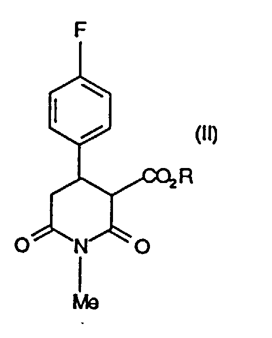 dans laquelle R représente un groupe alkyle en C₁ à C₆ ; en utilisant une enzyme consistant en une carboxylestérase,
(i) pour former un composé de formule (IIIA) 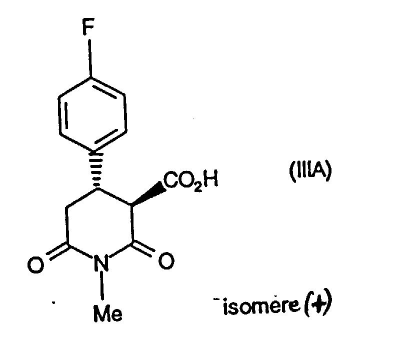 et en séparant ensuite le composé résultant de formule (IIIA) de l'isomère (-) restant de formule (II) ; ou
(ii) pour former un composé de formule (IIIb) : 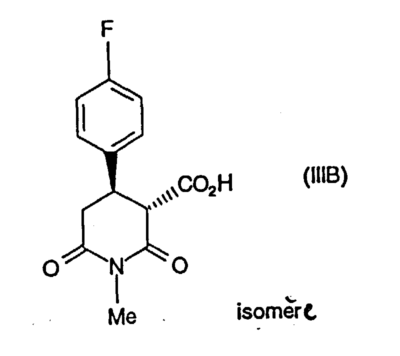 et en séparant ensuite le composé résultant de formule (IIIB) de l'isomère (+) restant de formule (II).

2. Procédé suivant la variante (i) dans la revendication 1, dans lequel la stéréosélectivité du procédé est telle que, à partir d'un mélange racémique d'un composé de formule (II), après l'action de l'enzyme consistant en une carboxyl-estérase, le rapport de l'isomère (-) à l'isomère (+) du composé de formule (II) soit supérieur à 60 %.

3. Procédé suivant la variante (ii) de la revendication 1, dans lequel la stéréosélectivité du procédé est telle que, à partir d'un mélange racémique d'un composé de formule (II), après l'action de l'enzyme consistant en une carboxyl-estérase, le rapport de l'isomère (+) à l'isomère (-) d'un composé de formule (II) soit supérieur à 60 %.

4. Procédé suivant l'une quelconque des revendications 1 à 3, qui est mis en oeuvre en dissolvant le composé (±), non séparé, de formule (II) dans un mélange d'eau et d'un solvant organique et en ajoutant l'enzyme consistant en carboxyl-estérase, puis en agitant le mélange résultant jusqu'à ce que la réaction parvienne à son terme.

5. Procédé suivant l'une quelconque des revendications 1 à 4, dans lequel la température pour la conduite de la réaction est comprise dans l'intervalle de 0 à 50°C.

6. Procédé suivant l'une quelconque des revendications 1 à 5, dans lequel le pH de la réaction est compris dans la plage de 4 à 8.

7. Procédé suivant l'une quelconque des revendications 1 à 6, dans lequel la variable R dans la formule (II) représente un groupe méthyle ou éthyle.

8. Procédé suivant la revendication 1, dans lequel l'enzyme consistant en une carboxyl-estérase est une estérase hépatique de porc ou une estérase hépatique bovine.

9. Procédé pour convertir ensuite un composé (-) de formule (II), préparé de la manière décrite dans la revendication 1, en paroxétine ou un de ses sels pharmaceutiquement acceptables et/ou de ses produits de solvatation pharmaceutiquement acceptables.

10. Procédé pour convertir ensuite un composé (-) de formule (III), préparé de la manière décrite dans la revendication 1, en paroxétine en le transformant tout d'abord en un composé (-) de formule (II), de la manière définie dans la revendication 1, en utilisant des techniques classiques d'estérification, avec ensuite une conversion en paroxétine ou un de ses sels pharmaceutiquement acceptables et/ou un de ses produits de solvatation pharmaceutiquement acceptables.

11. Procédé pour convertir ensuite un composé (-) de formule (III) directement en paroxétine en réduisant le groupe acide carboxylique en un groupe hydroxyméthyle et en réduisant les deux groupes céto dans le noyau pipéridine, puis en convertissant le pipéridine-carbinol résultant en paroxétine ou un de ses sels pharmaceutiquement acceptables et/ou un de ses produits de solvatation pharmaceutiquement acceptables.

12. Composé de formule (IIIA) ou (IIIB) suivant la revendication 1, ou un de sels ou hydrates.
